# EUROPÄISCHE PATENTANMELDUNG

(11) **EP 1 297 751 A1**
(43) Veröffentlichungstag der Anmeldung: **02.04.2003**
(21) Anmeldenummer: 01123561.1
(22) Anmeldetag: 01.10.2001
(51) Int. Cl.: A23K 1/16, A23K 1/175, A61K 35/78

(54) **Oral zu verabreichende Zubereitung**

(71) Anmelder: Bogar AG, 8002 Zürich (CH)
(72) Erfinder: Frater-Schröder, Marijke, 8400 Winterthur (CH); Frater, Georg, 8400 Winterthur (CH)
(74) Vertreter: Patentanwälte Schaad, Balass, Menzl & Partner AG

(57) **Zusammenfassung**

Die vorliegende Erfindung betrifft eine oral zu verabreichende Zubereitung, erhältlich durch
a) Zerkleinerung von Pflanzenmaterial,
b) Behandlung des zerkleinerten Pflanzenmaterials mit einem Extraktionsmittel ,
c) Durchmischen des zerkleinerten Pflanzenmaterials und des Extraktionsmittels,
d) Zugabe von porösen anorganischen Partikel zu der in Schritt c) erhaltenen Aufschlämmung,
e) Durchmischen der in Schritt d) erhaltenen Mischung
f) Trocknen der in Schritt e) erhaltenen Mischung.

## Beschreibung

Die vorliegende Erfindung betrifft oral zu verabreichende Zubereitung.

Der Einsatz von Ton in der Tierfutterindustrie ist schon seit längerem bekannt. Receuil de Médicine Vétérinaire 166 (1) (1990) S. 21-27 gibt einen Überblick über die Verwendung verschiedener Tonarten in der Futterindustrie.

Bentonit und Smektit werden als Futterzusatzstoffe bei Kälber und Schweinen verwendet und haben eine positive Wirkung bei Durchfall und bei der pH-Regulierung (W. Heinze und D. Oschika, Tierärztliche Umschau, 55, 621-627 und 678-683 (2000).

In WO 96/08168 wird ein Futterzusatz beschrieben, enthaltend eine Cholinverbindung, Fettsäuren und einen Träger, wobei der Träger aus Ton bestehen kann.

In WO 96/22028 wird ein Verfahren zur Herstellung eines Medikamentes für Tiere beschrieben, bei dem der Wirkstoff in einem kohäsiven Gel enthalten ist.

In EP 0 721 741 A1 wird die Verwendung von säureaktiviertem Montmorillonit-Ton bei Mycotoxin verseuchtem Futter beschrieben.

In FR 2 743 722 wird eine Zubereitung zur topischen Behandlung von Akne beschrieben, die Ton enthält.

In FR 2 703 242 wird eine Zubereitung enthaltend Ton und essentielle Öle für topische Anwendungen beschrieben.

Durch erblich bedingte Defekte oder durch ernährungs-, alterungs- oder umweltbedingte Faktoren kann die Funktion des unspezifischen Immunssystems und damit die Anfälligkeit von Tieren und Menschen auf virale oder bakterielle Infektionen wesentlich beeinflusst bzw. erhöht werden.

Die Aufgabe der vorliegenden Erfindung ist die Bereitstellung einer Zubereitung, welche eine homogene Wirkstoffverteilung, eine lange Wirkstoffstabilität und eine gute Bioverfügbarkeit aufweist.

Eine spezielle Aufgabe der vorliegenden Erfindung ist die Bereitstellung einer Zubereitung, die das unspezifische Immunssystem unterstützt und ausserdem eine lange Wirkstoffstabilität und eine gute Bioverfügbarkeit aufweist.

Die Aufgaben werden gelöst durch eine Zubereitung mit den Merkmalen von Anspruch 1. Weitere vorteilhafte Ausführungen der Erfindung ergeben sich aus den abhängigen Ansprüchen und der Beschreibung.

Die erfindungsgemässe oral zu verabreichende Zubereitung ist erhältlich durch
a) Zerkleinerung von Pflanzenmaterial,
b) Behandlung des zerkleinerten Pflanzenmaterials mit einem Extraktionsmittel,
c) Durchmischen des zerkleinerten Pflanzenmaterials und des Extraktionsmittels,
d) Zugabe von porösen anorganischen Partikeln zu der in Schritt c) erhaltenen Aufschlämmung,
e) Durchmischen der in Schritt d) erhaltenen Mischung
f) Trocknen der in Schritt e) erhaltenen Mischung.

Durch die Anwesenheit von zerkleinertem Pflanzenmaterial und den porösen anorganischen Partikeln enthält die erfindungsgemässe Zubereitung zwei Träger. Durch die Umverteilung einiger Stoffe von einem Träger, nämlich von dem zerkleinerten Pflanzenmaterial, auf den anderen Träger, d.h. auf die porösen anorganischen Partikel, sind beide Träger optimal beladen. Dadurch wird die erfindungsgemässe Zubereitung stabilisiert und die Stoffe sind besser bioverfügbar.

Die in der erfindungsgemässen Zubereitung enthaltenen porösen anorganischen Partikel enthalten vorzugsweise Ton oder Lehm. Besonders bevorzugt werden die anorganischen Partikel ausgewählt aus der Gruppe von Bentonit, Kaolinit, Smektit, Montmorillonit, Sepiolith und natürlicher Zeolithe. Der poröse Charakter dieser Partikel ermöglicht die Aufnahme der Extrakte des Pflanzenmaterials. Die anorganischen Partikel haben vorzugsweise eine Grösse bis zu 500 µm, vorzugsweise bis zu 250 µm und besonders bevorzugt bis zu 100 µm.

Unter dem Ausdruck Pflanzenmaterial werden in der vorliegenden Erfindung die ganzen Pflanzen oder Pflanzenteile wie Wurzeln, Stiele, Blätter oder Blüten verstanden. Das Pflanzenmaterial wird vorzugsweise ausgewählt aus der Gruppe der

| **Familie** | **Pflanzenname** | **Droge** | **Deutsche Bezeichnung** |
|---|---|---|---|
| Asteraceae | Achillea millefolium | Millefolii herba | Schafgarbenkraut |
| Apiaceae | Apium graveolens | Apii graveolentis radix | Selleriewurzel |
| | Boswellia serrata | Boswellia serrata Harz | Boswellia Harz |
| Papaveraceae | Chelidonium majus | Chelidonii herba | Schöllkraut |
| Apiaceae | Centella asiatica | Centellae asiaticae herba | Asiatisches Wassernabelkraut |
| Bruseraceae | Commiphora molmol | Myrrha | Myrrhe |
| Rosaceae | Crateaegus monogyna u./od. laevigata | Crateagi fructus | Weissdornfrüchte |
| Asteraceae | Cynara scolymus | Cynarae folium | Artischockenblätter |
| Asteraceae | Echinacea purpurea | Echinaceae purpureae radix | Rote Sonnenhautwurzel |
| Fabaceae | Foenum graecum | Foenugraeci semen | Bockshornsamen |
| Ginkgoaceae | Ginkgo biloba | Ginkgo biloba folium | Ginkoblätter |
| Hamamelidacaeae | Hamamelis virginiana | Hamamelidis folium | Hamamelisblätter |
| Hamamelidacaeae | Hamamelis virginiana | Hamamelidis cortex | Hamamelisrinde |
| Pedaliaceae | Harpagophytum procumbens | Harpagophyti radix | Teufelskrallenwurzel |
| Hypericaceae | Hypericum perforatum | Hyperici herba | Johanniskraut |
| Juglandaceae | Juglans regia | Juglandis folium | Walnussblätter |
| Lamiaceae | Leonurus cardiaca | Leonuri cardiaceae herba | Herzgespannkraut |
| Lamiaceae | Lyvopus europaeus | Lycopi herba | Wolfstrappkraut |
| Myrtaceae | Melaleuca alternifolia | Melaleucae aetheroleum | Melaleuca Oel |
| Fabaceae | Ononis spinosa | Ononidis radix (Hauhechelwurzel) | Hauhechelwurzel |
| Lamiaceae | Origanum vulgare | Origani herba | Dostenkraut |
| Passifloraceae | Passiflora incarnata | Passiflorae herba | Passionsblumenkraut |
| Piperaceae | Piper methysticum | Kava-Kava Rhizoma | Kava-Kave-Wurzel |
| Rosaceae | Potentila anserina | Anserinae Herba | Gänsefingerkraut |
| Primulaceae | Primula veris | Primulae radix | Primelwurzel oder Schlüssenblumen- |
| | | | wurzel |
| Fagaceae | Quercus robur | Quercus cortex | Eichenrinde |
| Krameriaceae | Krameria triandra | Ratanhiae radix | Ratanhiawurzel |
| Saxifragaceae | Ribes nigrum | Ribis nigri folium | Schwarze Johannisbeerblätter |
| Fabaceae | Robinia pseudacacia | Robinia fructus | Robinienbaum |
| Laminaceae | Salvia officinalis | Salivae folium | Salbeiblätter |
| Caesalipinia ceae | Sennae cassia | Cassia senuae | Sennablätter, Sennasamen |
| Asteraceae | Solidago virgaurea u./od. canadensis | Solidaginis herba | Goldrutenkraut |
| Asteraceae | Sylibum marianum | Cardui mariane fructus | Mariendistelfrüchte |
| Fabaceae | Trigonella foenum graecum | Foenugraeci semen | Bockshornsamen |
| Urticaceae | Urtica dioica | Urticae folium | Brennesselblätter |
| Urticaceae | Urtica dioica | Urticae radix | Brennesselwurzel |
| Ericaceae | Vaccinium vitis idaea | Vitis idaeae folium | Moosbeeren/ Preiselbeeren |
| Valerianaceae | Valeriana officinalis | Valerianae radix (Baldrian) | Baldrianwurzel |
| Violaceae | Viola tricolor | Violae tricoloris herba | Dreifaltigkeitskraut |
| Loranthaceae | Viscum album | Visci herba (Mistel) | Mistelkraut |
| Verbenaceae | Vitex agnus castus | Agni casti fructus | Mönchspfeffer |
| Lichenes | Cetraria islandica | Lichen islandicus | Isländisches Moos |
| Lichenes | Cetraria tenuifolia | Lichen islandicus | Isländisches Moos |
| Lichenes | Cetraroa ericetorum | Lichen islandicus | Isländisches Mooa |

Die oben erwähnten Pflanzen oder Pflanzenteile können alleine oder als Kombination mehrerer Pflanzen verwendet werden.

Vor der Verarbeitung wird das Pflanzenmaterial vorzugsweise getrocknet. Das Pflanzenmaterial wird in Teilschritt a) des oben beschriebenen Verfahrens mittels Mahlen, Schneiden, Rupfen oder einem anderen geeigneten Verfahren zerkleinert. Das zerkleinerte Pflanzenmaterial hat eine Grösse bis zu 1000 µm, vorzugsweise eine Grösse bis zu 710 µm. Die Maximalverteilung des zerkleinerten Pflanzenmaterials ist vorzugsweise im Bereich von 200 bis 300 µm, vorzugsweise bei 250 µm. Damit weist das zerkleinerte Pflanzenmaterial vorzugsweise ungefähr die gleiche Grösse auf wie die porösen anorganischen Partikel.

Als Extraktionsmittel kommen sämtliche dem Fachmann bekannten organischen Lösungsmittel, Mischungen von Lösungsmitteln und Mischungen von Lösungsmitteln mit Wasser in Frage, die die Fähigkeit haben, Stoffe aus dem Pflanzenmaterial herauszulösen. Bevorzugt sind Alkohole, besonders bevorzugt sind jedoch reiner Ethanol oder Ethanol/Wassermischungen in Verhältnissen von 75:25 bis zu 99:1%. Weitere geeignete Extraktionsmittel sind z.B. Methanol oder Methylenchlorid.

Das zerkleinerte Pflanzenmaterial wird in Teilschritt b) in das Extraktionsmittel gegeben. Durch das innige Mischen des zerkleinerten Pflanzenmaterials und des Extraktionsmittels werden dem Pflanzenmaterial Komponenten in Teilschritt c) entzogen. Die anorganischen Partikel werden anschliessend zu der in Teilschritt c) erhaltenen Aufschlämmung gegeben. Die porösen anorganischen Partikel, das Pflanzenmaterial und dessen Extrakte werden anschliessend durchmischt, wobei eine gleichmässige Mischung entsteht. Die obengenannte Mischung der porösen anorganischen Partikel, des Pflanzenmaterials und dessen Extrakte wird nach dem Mischen getrocknet. Dabei liegt der Feuchtigkeitsgehalt vorzugsweise unterhalb 5% bezogen auf das Gesamtgewicht der Zubereitung liegen. Der Ethanolgehalt der erfindungsgemässen Zubereitung sollte möglichst tief sein und liegt vorzugsweise unterhalb 0.5% bezogen auf das Gesamtgewicht der Zubereitung. Die erfindungsgemässe Zubereitung liegt vorzugsweise als Pulver vor. Andere Extraktionsmittel, wie Methanol oder Methylenchlorid müssen weitgehend entfernt werden.

Das Verhältnis des porösen, anorganischen Materials zu dem Pflanzenmaterial in der erfindungsgemässen Zubereitung ist im Bereich von 0.33:1 bis 10:1, bevorzugt im Bereich von 1:1 bis 5:1 und besonders bevorzugt im Bereich von 2:1 bis 3:1.

Durch die Verwendung von Ethanol bzw. eines Ethanol Wassergemischs in Teilschritt b) des oben beschriebenen Verfahrens wird auch eine antiseptische Wirkung erzielt. Dadurch und durch den Wasserentzug wird die Langzeitstabilität des Produktes in trockenem Zustand zusätzlich erhöht, ohne dass zeitaufwendige oder teure Stabilisierungsmassnahmen ergriffen werden müssen. Die erfindungsgemässe Zubereitung hat eine Stabilität von mindestens 2 Jahren.

Durch das Vorhandensein sowohl des zerkleinerten Pflanzenmaterials als auch dessen Extrakte in der erfindungsgemässen Zubereitung gehen keine Inhaltsstoffe des Pflanzenmaterials verloren. Ausserdem weist die erfindungsgemässe Zubereitung eine ausnehmend gute Bioverfügbarkeit auf, da sonst für den Körper nur schwer zugängliche Komponenten des Pflanzenmaterials durch das anorganische poröse Material besser verfügbar gemacht werden. Gleichzeitig können andere Komponenten in dem Pflanzenmaterial wiederum die Resorption gewisser anderer Komponenten fördern oder hemmen. Durch das Zusammenspiel der porösen anorganischen Partikel, des zerkleinerten Pflanzenmaterials und dessen Extrakte wird eine ausnehmend gute therapeutische Wirksamkeit erzielt.

Beispielsweise hat die in Beispiel 5 beschriebene Zubereitung eine ausnehmend positive Wirkung sowohl auf das unspezifische Immunssystem als auch auf den Verlauf viraler und bakterieller Infekte hat. Dank dem komplexen Zusammenspiel aller Inhaltsstoffe dieser erfindungsgemässen Zubereitung entfaltet sich eine breite multifunktionale, therapeutische Wirksamkeit was zur Folge hat, dass eine Gesamtwirkung erzielt wird, die höher ist als die Summe aller Einzelwirkungen, d.h. dass synergistische Effekte vorliegen.

Die überraschend gute Wirkung der in Beispiel 5 beschriebenen Zubereitung ergibt sich unter anderem durch die gute Bioverfügbarkeit der einzelnen Komponenten und dadurch, dass die Wirkstoff-Komponenten an unterschiedlichen Stellen des Heilungsprozesses und des Abwehrsystems einsetzen. Diese erfindungsgemässe Zubereitung fördert auf diese Weise sowohl bei geschwächtem als auch bei intaktem Immunsystem die mikrobielle Abwehr. Das Zusammenwirken der porösen anorganischen Partikel, des Pflanzenmaterials und dessen Extrakte hat einen signifikant positiven und synergistischen Effekt auf das unspezifische Abwehrsystem und auf den Heilungsprozess bei hartnäckigen und/oder chronischen Atemwegerkrankungen.

Diese erfindungsgemässe Zubereitung, die Echinacea purpurea enthält, eignet sich insbesondere zur Stärkung des körpereigenen Immunsystems und unterstützt die Heilung von hartnäckigen und/oder chronischen Atemwegserkrankungen. Ausserdem eignet es sich für die Behandlung von saison- und altersbedingten Schwächezuständen des Immunsystems, saisonalen Atemwegserkrankungen wie Erkältungen, Zwingerhusten, Bronchitis, Phargynitis, Tonsillitis. Nach Einnahme dieser erfindungsgemässen Zubereitung konnten keine Nebenwirkungen beobachtet werden.

Vorzugsweise wird einem Tier oder einem Menschen 1 Gramm der in Beispiel 5 beschriebenen Zubereitung pro 10 kg Körpergewicht in feuchtem Futter beziehungsweise Essen verabreicht. Im Falle einer Behandlung der obengenannten Erkrankungen sollte diese erfindungsgemässe Zubereitung während mindestens zwei Wochen vorzugsweise jedoch vier bis acht Wochen regelmässig eingenommen werden. Die erfindungsgemässe Zubereitung kann jedoch auch prophylaktisch verabreicht werden und wird damit für ein verbessertes allgemeines Wohlbefinden sorgen.

Mit anderem Pflanzenmaterial können beispielsweise dyseptische Beschwerden oder bei Magen-Darm- und Gallenstörungen behandelt werden. Damit stellt die erfindungsgemässe Zubereitung ein wirksames Mittel dar.

Die erfindungsgemässe Zubereitung wird vorzugsweise Säugetieren und aquatischen Tieren verfüttert. Besonders bevorzugt sind Haustieren wie Hunde, Katzen, Fische, Kaninchen, Hasen und Vögel, oder Zuchttiere wie Kälber, Rinder, Kühe, Pferde, Schafe, Schweine, Hühner, Krebse und Fische. Die erfindungsgemässe Zubereitung steigert auch das allgemeine Wohlbefinden von Menschen nach oraler Einnahme.

Die erfindungsgemässe Zubereitung wird vorzugsweise als Pulver oder als Granulat direkt unter das Futter gemischt werden oder aber bereits schon bei der Herstellung in das Futter eingearbeitet werden. Dabei sind eine Vielzahl von verschiedenen Verabreichungsmöglichkeiten möglich wie zum Beispiel Trockenfutter, Kekse etc. Alternativ kann die erfindungsgemässe Zubereitung auch in einen Lebensmittelzusatz eingearbeitet werden oder als solcher verwendet werden. Die erfindungsgemässe Zubereitung kann neben den oben erwähnten Inhaltsstoffen noch die in galenischen Zubereitung üblichen Zusätze enthalten.

Die nachfolgenden Beispiele dienen dazu die Erfindung zu erläutern, ohne deren Umfang einzuschränken.

### Beispiel 1

Eingesetzte Rohstoffe
Hyperici herba: (gemahlen auf ≤ 0.25 mm)
Sepiolith
Ethanol 96 %

300g gemahlenes Pflanzenmaterial werden in Behälter vorgelegt und mit 450 g Ethanol 96% versetzt. Die Mischung wird 1 h gerührt. Anschliessend 900 g Sepiolith dazugeben und gut mischen. Die Mischung 48 h im Vakuumtrockenschrank bei 40° C trocknen. Durch 0.355 mm Sieb sieben und nochmals gut mischen.
Ausbeute: 1.12 kg
Indikation: antiviral

In Analogie zu dem vorstehenden Beispiel wurden die in der vorstehenden Tabelle aufgeführten Pflanzen anstelle von Hyperici herba eingesetzt und analog weiterverarbeitet.

### Beispiel 2

Eingesetzte Rohstoffe
Quercus cortex: (gemahlen a auf ≤ 1 mm)
Sepiolith
Methanol 95%

300g gemahlenes Pflanzenmaterial werden in Behälter vorgelegt und mit 450 g Methanol 95 % versetzt. Die Mischung wird 1 h gerührt. Anschliessend 600 g Sepiolith zugeben und gut mischen. Die Mischung 48 h im Vakuumtrockenschrank bei 40° C trocknen. Durch 0.71 mm Sieb sieben und nochmals gut mischen.
Ausbeute: 820 g
Indikation: Adstringenz bei Durchfall

### Beispiel 3

Eingesetzte Rohstoffe
Salvis officinalis folium: (gemahlen auf ≤ 1mm)
Sepiolith
Ethanol 96%

300g gemahlenes Pflanzenmaterial werden in Behälter vorgelegt und mit 450 g Ethanol 96% versetzt. Die Mischung wird 1 h gerührt. Anschliessend 300g Sepiolith dazugegeben und gut mischen. Die Mischung 48 h im Vakuumtrockenschrank bei 40° C trocknen. Durch 0.71 mm Sieb sieben und nochmals gut mischen.
Ausbeute: 545 g
Indikation: dyspeptische Beschwerden, Magen-Darm-Galle-Störung.

### Beispiel 4

Eingesetzte Rohstoffe
Cynarae folium
Sepiolith
Ethanol 96 %
Das 1.25 kg Pflanzenpulver wird im 61 Becken vorgelegt und mit 1.5 l Ethanol 75° C übergossen. Nach dem Mischen ist die Temperatur ca. 46° C. Die Mischung mit Folie zudecken und 1 h stehen lassen. Anschliessend 1.25 kg Sepiolith zugeben und sehr gut durchmischen. Das Produkt wird auf einer Polyethylenfolie verteilt und bei Raumtemperatur unter Abzug 20 h getrocknet. Das ganze wird durch ein 1mm Sieb gesiebt und im Vakuumtrockenschrank bei max. 45°C für 30 h getrocknet.
Ausbeute 2.41 kg
Indikation: Cholacogum, dyspeptische Beschwerden, k Magen-Darm-Galle-Störungen

### Beispiel 5

Eingesetzte Rohstoffe
Echinacea purpurea
Sepiolith
Ethanol 96 %

Das 1.25 kg Pflanzenpulver wird im 61 Becken vorgelegt und mit 1.5 l Ethanol 75° C übergossen. Nach dem Mischen ist die Temperatur ca. 46° C. Die Mischung mit Folie zudecken und 1 h stehen lassen. Anschliessend 2.5 kg Sepiolith zugeben und sehr gut durchmischen. Das Produkt wird auf einer Polyethylenfolie verteilt und bei Raumtemperatur unter Abzug 20 h getrocknet. Das ganze wird durch ein 1mm Sieb gesiebt und im Vakuumtrockenschrank bei max. 45°C für 30 h getrocknet.
Ausbeute 3.5 kg
Indikation: allgemeine Immunstimulanz, Zwingerhusten

Die obengenannte Zubereitung wurde nach dem Pharmakopöe unter anderem mit den Leitsubstanzen Dodeca (2E, 4E, 8Z, 10E)-tetraencarbonsäureisobutylamid und Zichoriensäure auf ihre Stabilität überprüft.

### Beispiel 6: Klinische Studie

Eine offene multizentrische kontrollierte klinische Studie wurde durchgeführt, um Wirksamkeit und Verträglichkeit in Beispiel 5 beschriebenen Zubereitung bei Hunden bei der Behandlung chronischer und/oder rezidivierender Infektionen der Atemwege (Pharyngitis/Tonsillitis, Bronchitis, Zwingerhusten) und/oder bei unterentwickelten Jungtieren nachzuweisen. Jedes Tier diente als sein eigenes Kontrollelement bei den chronischen Erkrankungen. Der chronische Zustand gemäss Krankengeschichte wurde im CRF (Prüfbericht) festgehalten.

In die Studie wurden einundvierzig (41) Hunde aufgenommen. Dies war die Intention to Treat (ITT)-Population. Bei neununddreissig (39) Hunden wurde die.Behandlung nach vier Wochen hinsichtlich Verträglichkeit und Wirksamkeit beurteilt, bei achtunddreissig (38) Hunden auch nach acht Wochen Behandlungsdauer. Diese Hunde bildeten die akzeptierbare Patientenpopulation (PP). Die Hunde wurden durch sechs in der Schweiz praktizierende Tierärzte mit schriftlicher Einverständniserklärung der Tierbesitzer ausgewählt. Die Auswahl basierte auf der Erfüllung von Ein- und Ausschlusskriterien sowie einer klinischen Untersuchung gemäss Studienprüfplan. Nur ein Hund konnte die Studie nicht abschliessen, da er ohne Zusammenhang mit der Studie ein muskuloskelettales Krankheitsbild entwickelte, das eine Therapie mit Steroiden erforderte.

Einundvierzig (41) Hunde, bei denen eine oder mehrere der folgenden chronischen und/oder rezidivierenden Erkrankungen der Atemwege diagnostiziert wurden, wurden in die Studie aufgenommen: Zwingerhusten, Bronchitis und Pharyngitis/Tonsillitis. Obschon rezidivierende Pneumonie ebenfalls ein Einschlusskriterium darstellte, wurden keine Hunde mit diesem Krankheitsbild aufgenommen. Im Studienprotokoll wird ein chronisches Krankheitsbild definiert als Erkrankung, von der nicht erwartet wird, dass sie sich unbehandelt innert acht Wochen verbessere. Drei unterentwickelte Junghunde wurden ebenfalls in die Studie aufgenommen. Sämtliche Tiere wurden vom Prüftierarzt vor der Therapie sowie nach vier und acht Wochen Behandlungsdauer untersucht. 30 Tiere wurden zusätzlich nach 15 ± 3 Tagen untersucht. Bei jeder Untersuchung wurden Auftreten und Schweregrad folgender klinischer Parameter und Symptome festgehalten: Körpergewicht, Temperatur (rektal), Fellzustand, Appetit, Lethargie, Augenausfluss (klar und eitrig), Nasenausfluss (klar und eitrig), Zyanose, Vergrösserung der Lymphknoten, Dehydrierung, Husten, Dyspnöe und Lungengeräusche (feucht und trocken). Blutproben für hämatologische und klinischchemische Untersuchungen wurden vor Therapiebeginn und nach acht Wochen Behandlungsdauer entnommen. Der Tierbesitzer verabreichte die erfindungsgemässe Zubereitung während acht Wochen in einer Dosierung von 0.1g/k Körpergewicht mit feuchtem Futter vermischt. Die Medikamentenabgabe wurde durch den Tierbesitzer in einem "Tierbesitzer-Tagebuch" festgehalten.

Der Prüftierarzt beurteilte anhand der Symptomänderungen global die Wirksamkeit der Behandlung für jeden Hund nach vier und acht Wochen als "sehr gut", "gut", "mässig" oder "ungenügend". Die sekundären Wirksamkeitsparameter wurde durch Beurteilung des Schweregrades der Symptome Fellzustand, Appetit, Lethargie, Augenausfluss (klar und eitrig), Nasenausfluss (klar und eitrig), Zyanose, Vergrösserung der Lymphknoten, Dehydrierung, Husten, Dyspnöe und Lungengeräusche (feucht und trocken) vor und nach der Behandlung mit der erfindungsgemässen Zubereitung beurteilt. Der Tierarzt beurteilte global die Verträglichkeit der Behandlung als "sehr gut", "gut", "mässig" oder "ungenügend" für jeden Hund nach vier und acht Wochen Behandlungsdauer anhand von Auftreten und Natur aller unerwünschten Wirkungen.

### Ergebnisse

Die Wirksamkeitsdaten von Hunden mit Pharyngitis/Tonsillitis, Bronchitis, Zwingerhusten und/oder unterentwickelten Jungtieren wurden mit dem SAS-Statistikpaket Version 6.12 analysiert. Von den 41 in die Studie aufgenommenen Hunden wurden die Daten von 39 Tieren analysiert, da zwei Hunde (Nummern 8 und 39) während der Studiendauer eine Behandlung mit Antibiotika erforderten und deshalb von der Auswertung ausgeschlossen wurden. Nach acht Wochen Behandlungsdauer wurden noch die Daten von 38 Hunden analysiert, da Hund Nr. 29 infolge eines nicht mit der Studie in Beziehung stehenden klinischen Krankheitsbildes, das eine Behandlung mit Steroiden erforderte, aus der Studie ausgeschlossen wurde. Sowohl nach vier wie nach acht Wochen Behandlungsdauer wurde eine statistisch signifikante Wirksamkeit nachgewiesen. In 92.3% der Fälle (36/39) wurde die Wirksamkeit nach vier Wochen Behandlungsdauer mit der erfindungsgemässen Zubereitung bei einem 95%-Vertrauensintervall zwischen 88% und 102% als "gut" oder "sehr gut" beurteilt. Die globale Wirksamkeit nach acht Wochen Behandlungsdauer wurde in 94.8% der Fälle (36/38) bei einem 95%-Vertrauensintervall zwischen 88% und 102% als "gut" oder "sehr gut" beurteilt.

Mit Hilfe des Bowker-Tests konnte eine hoch signifikante (p < 0.01) Reduktion des Schweregrades für sämtliche der folgenden Haupt-Symptome dieser Studie sowohl nach vier wie nach acht Wochen Behandlungsdauer nachgewiesen werden: Fellzustand, Appetit, Lethargie, Augenausfluss (eitrig), Nasenausfluss (klar), Vergrösserung der Lymphknoten, Husten (trocken), Dyspnöe und Lungengeräusche (trocken). Bei klarem Augenausfluss wurde nach vier (p < 0.04) und acht Wochen (p < 0.01) Behandlungsdauer eine signifikante Reduktion festgestellt. Eine statistisch signifikante Reduktion der (rektal gemessenen) Körpertemperatur wurde sowohl nach vier wie nach acht Wochen Behandlungsdauer nachgewiesen.

Die primäre Beurteilung der Verträglichkeit wurde auf Grundlage des Auftretens und der Natur unerwünschter Wirkungen vorgenommen. Im Rahmen dieser Studie sind keine Tiere gestorben. Nur bei einem Hund (1/39) wurde eine unerwünschte Wirkung festgestellt. Bei diesem Tier (Nr. 8) wurden Bronchitis und Pharyngitis/Tonsillitis diagnostiziert. Unter der Behandlung mit der erfindungsgemässen Zubereitung trat anfangs eine Besserung auf. An Tag 26 verschlimmerte sich der Husten stark und wurde als schwerwiegende unerwünschte Wirkung festgehalten. Nach Ansicht des Tierarztes war durch Superinfektion Zwingerhusten aufgetreten. Dem Tier wurde während acht Tagen ein Antibiotikum (SYNULOX®, Pfizer Ltd) verschrieben. Die Behandlung mit der erfindungsgemässen Zubereitung wurde in diesem Zeitraum fortgesetzt. Nach der Verabreichung des Antibiotikums verbesserte sich der Zustand des Hundes rasch. Obschon dieses Tier aufgrund der gleichzeitigen Behandlung mit Antibiotika aus der Studie ausgeschlossen hätte werden sollen, bewilligte der Studienmonitor sein Verbleiben in der Studie (Abweichung 3). Im Rahmen der achtwöchigen Behandlung traten bei diesem Tier keine weiteren unerwünschten Wirkungen auf. Das Auftreten der beschriebenen unerwünschten Wirkung kann nicht klar auf die Behandlung mit der erfindungsgemässen Zubereitung zurückgeführt werden.

Die systemische Verträglichkeit der erfindungsgemässen Zubereitung wurde in den hämatologischen und klinischchemischen Befunden vor und nach acht Wochen Behandlung nachgewiesen. Nach vier Wochen wurde die globale Verträglichkeit des Präparates in 100% der Fälle (39/39) als "gut" oder "sehr gut" beurteilt, nach acht Wochen in 100% der Fälle (38/38) als "gut" oder "sehr gut".

### Schlussfolgerungen

Diese Studie hat gezeigt, dass die erfindungsgemässe Zubereitung, oral verabreicht in einer Dosis von 0.1g/kg Körpergewicht während vier bis acht Wochen, für Hunde verträglich und wirksam bei der Behandlung chronischer und/oder rezidivierender Infektionen der Atemwege einschliesslich Pharyngitis/Tonsillitis, Bronchitis und Zwingerhusten ist. Nach vier Wochen Behandlungsdauer mit der erfindungsgemässen Zubereitung wurde eine statistisch signifikante (p < 0.01) Reduktion des Schweregrades der Symptomatik oder Symptomelimination chronischer und/oder rezidivierender Infektionen der Atemwege nachgewiesen, die auch nach acht Wochen noch klar vorhanden war.

## Patentansprüche

1. Oral zu verabreichende Zubereitung, erhältlich durch
a) Zerkleinerung von Pflanzenmaterial,
b) Behandlung des zerkleinerten Pflanzenmaterials mit einem Extraktionsmittel,
c) Durchmischen des zerkleinerten Pflanzenmaterials und des Extraktionsmittels,
d) Zugabe von porösen anorganischen Partikel zu der in Schritt c) erhaltenen Aufschlämmung,
e) Durchmischen der in Schritt d) erhaltenen Mischung
f) Trocknen der in Schritt e) erhaltenen Mischung.

2. Zubereitung nach Anspruch 1, in der die porösen anorganischen Partikel Ton enthalten.

3. Zubereitung nach Anspruch 1, in der die anorganischen Partikeln ausgewählt sind aus der Gruppe der Silikate.

4. Zubereitung nach Anspruch 3, **dadurch gekennzeichnet, dass** die anorganischen Partikel ausgewählt sind aus der Gruppe von Bentonit, Smektit, Montmorillonit, Kaolinit, Sepiolith und natürliche Zeolithe.

5. Zubereitung nach einem der vorangehenden Ansprüche, in der das Pflanzenmaterial ausgewählt ist aus der Gruppe der *Echinacea purpurea, Crateaegus monogyna und*/*oder laevigata, Melaleuca alternifolia, Solidago virgaurea und*/*oder canadensis, Ononis spinosa*, *Boswellia serrata, Piper methysticum, Cynara scolymus, Trigonella foenum graecum, Passiflora incarnata, Juglans regia, Achillea millefolium, Sennae cassia, Vitex agnus castus, Harpagophytum procumbens, Viscum album, Robinia pseudacacia, Valeriana officinalis, Hamamelis virginiana, Quercus robur, Potentila anserina, Hypericum perforatum, Salvia officinalis, Vaccinium vitis idaea, Cetraria islandica, Cetraria tenuifolia, Cetraria ericetorum und Sylibum marianum*.

6. Zubereitung nach Anspruch 5, enthaltend Echinacea purpurea.

7. Futterzusatz, enthaltend die Zubereitung gemäss einem der vorangehenden Ansprüche.

8. Futter, enthaltend die Zubereitung gemäss einem der Ansprüche 1 bis 6.

9. Verfahren zur Herstellung einer oral zu verabreichenden Zubereitung, durch
a) Zerkleinerung von Pflanzenmaterial,
b) Behandlung des zerkleinerten Pflanzenmaterials mit einem Extraktionsmittel
c) Durchmischen des zerkleinerten Pflanzenmaterials und des Extraktionsmittels,
d) Zugabe von porösen anorganischen Partikel zu der in Schritt c) erhaltenen Aufschlämmung
e) Durchmischen der in Schritt d) erhaltenen Mischung,
f) Trocknen der in Schritt e) erhaltenen Mischung.

10. Verfahren nach Anspruch 9 durch
a) Zerkleinerung von Echinacea purpurea,
b) Behandlung der zerkleinerterten Echinacea purpurea mit einem Alkohol,
c) Durchmischen der zerkleinerterten Echinacea purpurea und des Extraktionsmittels,
d) Zugabe von Sepiolith zu der in Schritt c) erhaltenen Aufschlämmung,
e) Durchmischen der in Schritt d) erhaltenen Mischung
f) Trocknen des so erhaltenen Mischung.
